# EUROPÄISCHE PATENTSCHRIFT

(11) **EP 1 312 060 B1**
(45) Veröffentlichungstag und Bekanntmachung des Hinweises auf die Patenterteilung: **26.10.2005**
(21) Anmeldenummer: 00991255.1
(22) Anmeldetag: 22.12.2000
(51) Int. Cl.: G08B 21/00, G01N 27/327, G01N 33/48, A61B 5/00

(54) **VORRICHTUNG, VERFAHREN UND VERWENDUNG EINER GERUCHSSENSOREINHEIT ZUR UNTERSUCHUNG UND/ODER PROTOKOLLIERUNG**
DEVICE, METHOD AND USE OF AN ODOR DETECTION UNIT FOR EXAMINING AND/OR RECORDING
DISPOSITIF, PROCEDE ET UTILISATION D'UNE UNITE DE DETECTION OLFACTIVE S'UTILISANT A DES FINS D'EXAMEN ET/OU DE PROTOCOLE

(30) Priorität: 23.12.1999 DE 19962808
(43) Veröffentlichungstag der Anmeldung: 21.05.2003
(73) Patentinhaber: Thomas Hilfen HILBEG GmbH & Co. Kommanditgesellschaft, 27432 Bremervörde (DE)
(72) Erfinder: JANSEN, Klaus, 21614 Buxtehude (DE)
(74) Vertreter: Möller, Friedrich
(86) Internationale Anmeldenummer: PCT/EP2000/013150
(87) Internationale Veröffentlichungsnummer: WO 2001/048473

(56) Entgegenhaltungen:
- DE-A- 19 730 733
- DICKINSON TODD A ET AL: "Current trends in 'artificial-nose' technology." TRENDS IN BIOTECHNOLOGY, Bd. 16, Nr. 6, Juni 1998 (1998-06), Seiten 250-258, XP004121066 ISSN: 0167-7799

## Beschreibung

Die Erfindung betrifft eine Vorrichtung zur Untersuchung und/oder Protokollierung von menschlichen Erkrankungen, Körperzuständen und/oder physiologischen Prozessen einer Person gemäß dem Oberbegriff des Anspruchs 1, ein Verfahren zur Untersuchung und/oder Protokollierung von menschlichen Erkrankungen, Körperzuständen und/oder physiologischen Prozessen einer Person gemäß dem Oberbegriff des Anspruchs 8 und bevorzugte Verwendungen der Vorrichtung.

Im Bereich der Pflege, insbesondere Altenpflege, besteht zum Teil ein erheblicher Mangel an Arbeitskräften. Dies führt dazu, dass die in diesem Bereich tätigen Arbeitskräfte häufig überlastet und unmotiviert sind. Dadurch werden erforderliche Pflegemaßnahmen nicht oder nicht ordnungsgemäß durchgeführt. Es besteht daher ein Wunsch zur Überwachung der durchgeführten Pflegemaßnahmen. Eine Überwachung mittels Videokameras ist aus datenschutzrechtlichen Gründen problematisch. Eine Befragung der Pflegebedürftigen ist hingegen auch mit Problemen behaftet, da diese entweder nicht mehr in der Lage sind, gezielt und objektiv zu antworten, oder wegen ihrer Abhängigkeit von den Pflegepersonen unwahrheitsgemäß antworten.

Aus der DE 197 30 733 A ist ein Gerät zur Patientenüberwachung bekannt, das einen Sensor aufweist, der eine "elektronische Nase" darstellt. Der Sensor ist gezielt über dem Gesicht eines auf einem Tisch liegenden Patienten angeordnet und so ausgebildet, das er im Falle eines Erbrechens des Patienten einen Alarm gibt. Abgesehen davon, dass die Anordnung des Sensors über dem Gesicht des Patienten stört, kann der Sensor nur das Erbrechen des Patienten detektieren; eine umfassende Überwachung des Patienten ist jedoch mit dem bekannten Gerät nicht möglich.

Aus dem Aufsatz "Current trends in artifical-nose technology" von Todd Dickinson et al (Trends in Biotechnology, Bd. 16, Nr. 6, Juni 1998, Seiten 250-258) ist eine Übersicht der Entwicklung auf dem Gebiet "künstlicher Nasen" bekannt und es sind verschiedene Anwendungen in der medizinischen Diagnostik zusammengestellt. Die Überwachung des Gesundheitszustands einer Person anhand spezifischer Gerüche des Körpers derselben geht aus diesem Aufsatz nicht hervor.

Der Erfindung liegt daher das technische Problem zugrunde, die Beobachtung und/oder Versorgung von Personen (Probanden oder Patienten) zu verbessern.

Eine Vorrichtung zur Lösung dieses Problems weist die Merkmale des Anspruchs 1 auf.

Das eingangs genannte Problem wird des Weiteren durch ein Verfahren mit den Maßnahmen des Anspruchs 8 gelöst.

Der Erfindung liegt die Erkenntnis zugrunde, dass ein menschlicher Körper je nach Körperzustand unterschiedliche Geruchsstoffe aussondert, wobei diese Geruchsstoffe von unterschiedlichen Körperstellen stammen können. Deswegen wird der Geruch des ganzen Körpers oder mindestens eines relevanten Großteils des Körpers ermittelt. Diese Geruchsstoffe liegen bereits bei geringsten Zustandsänderungen des Körpers vor, jedoch zum Teil in einer extrem niedrigen Konzentration, so dass sie vom Menschen praktisch nicht wahrnehmbar sind.

Gemäß der Erfindung wird daher eine empfindliche "künstliche Nase", nämlich eine Geruchssensoreinheit zum Erfassen und Untersuchen der Geruchsstoffe eingesetzt. Anhand der gemessenen Geruchsstoffe kann man - nach einer anschließenden, eingehenden Bewertung - auf einen Körperzustand schließen. Insbesondere lassen sich jedoch auch anhand von Konzentrationsänderungen von (verschiedenen) Geruchsstoffen Rückschlüsse auf Körperzustände bzw. deren Veränderungen ziehen. Insgesamt gewinnt man anhand der gemessenen Geruchsstoffe bzw. der gemessenen Expression olfaktorischer Informationen Kenntnisse über genetische und/oder physiologische Zustände sowie immunologische Vorgänge im Organismus. Hierdurch eröffnen sich zahlreiche Anwendungsgebiete, insbesondere zur Untersuchung und Überwachung von pflegebedürftigen Menschen, Patienten oder allgemein Probanden.

Besonders wird die Erfindung auch zur Protokollierung einer Pflegebehandlung oder eines beliebigen Krankheitsverlaufs eingesetzt. Anhand der Veränderung der Geruchsstoffkonzentrationen, lassen sich Rückschlüsse auf eine eventuelle Besserung, Verschlechterung oder einen gleichbleibenden Zustand schließen.

Ferner kann die Erfindung auch vorteilhaft zur Bestimmung von Schlafstadien sowie weiteren physiologischen Prozessen während des Schlafes genutzt werden. Hierdurch können neue Erkenntnisse über Schlafgewohnheiten im allgemeinen sowie die besondere Schlafzyklen von Probanden untersucht werden.

Darüber hinaus kann die Erfindung auch zur Beobachtung eines eventuellen Heilungsprozesses einer Erkrankung, beispielsweise eines Geschwürs, insbesondere Dekubitusgeschwürs, eingesetzt werden. Es können auch anderen Entzündungen im kutanen und/oder subkutanen Bereich und deren Entstehung mittels der Erfindung beobachtet werden. Außerdem kann die Erfindung zur Früherkennung von Trombose oder dergleichen Erkrankungen eingesetzt werden. Man erhält somit die Möglichkeit geeignete Prophylaxe- und Therapiemaßnahmen gezielt einzuleiten. Eine eventuelle Therapie kann somit an den jeweiligen Heilungsprozess angepasst, d.h. phasenoptimiert werden. Die Anzeige der gemessenen Geruchsstoffe liefert dabei ein Zwischenergebnis, das für die Optimierung und auch in einer Diagnose verwertbar ist.

Bevorzugt weist eine Sensoreinheit ein Sensorarray, insbesondere ein Geruchssensorarray, aus einer Mehrzahl einzelner Gassensoren auf, die gegebenenfalls unterschiedlich auf spezifische Geruchsstoffe reagieren. So erhält man bei Untersuchung einer Geruchsprobe eine der Anzahl der Gassensoren entsprechende Anzahl von Sensorsignalen. Durch eine flächige Verteilung der Geruchssensoren lässt sich der erforderliche, vorzugsweise gesamte, Körperoberflächengeruch ermitteln und auswerten.

Bevorzugt wird das bzw. werden die Sensorsignale der Geruchssensoreinheit fortlaufend aufgezeichnet, um Veränderungen erkennen zu können. Durch den weiter bevorzugten Einsatz mehrerer Geruchssensoreinheiten lassen sich auch lokale oder globale spezifische Veränderungen des Probandengeruchs und/oder die Einstellung eines spezifischen Probandengeruchs beobachten.

Die erhaltenen Informationen lassen sich beispielsweise bei der Entstehung eines Dekubitusgeschwürs - das insbesondere bei mangelnder Pflege entstehen kann - oder auch zur Trombosefrüherkennung nutzen. Dadurch können auf vorteilhafte Weise Gegenmaßnahmen eingeleitet werden. Die Detektion bereits geringster Geruchsstoffe ermöglicht somit eine Früherkennung von zum Beispiel Dekubitus.

Des weiteren sind auch dermale Irritationen erkennbar. Bevorzugt kann daher bei Überschreiten bzw. Erreichen bestimmter Grenzwerte ein spezifisches Alarmsignal dank der Erfindung ausgelöst werden, um weitere Untersuchungsmaßnahmen oder ggf. Behandlungsmaßnahmen einzuleiten.

Weitere Einzelheiten der Erfindung ergeben sich aus den Unteransprüchen sowie aus dem nachfolgend erläuterten Ausführungsbeispiel eines Monitoringsystems mit einer Geruchssensoreinheit.

Gemäß einem Ausführungsbeispiel wird eine elektronische Sensoreinheit verwendet. Sie verfügt über geringe Abmessungen und kann schnell, d.h. innerhalb weniger Sekunden oder Minuten, Ergebnisse liefern. Die Sensoreinheit weist eine Gasprobennahmeeinheit, ein Sensorarray und eine Auswerteeinheit auf. Damit ist der Gesamtkörpergeruch von Personen zur Prophylaxe und/oder Früherkennung von Krankheiten heranziehbar.

Das Sensorarray besteht aus einer Mehrzahl von Halbleiter-Gassensoren und zwar beispielsweise aus zehn derartigen Gassensoren. Die Gassensoren sind derart räumlich verteilt angeordnet, dass sie Gerüche von verschiedenen Stellen des Körpers detektieren. Insbesondere sind die Gassensoren zur Ermittlung von Gerüchen des ganzen Körpers angeordnet, indem sie den ganzen Körper auf Gerüche abscannen. Diese Halbleitergassensoren sind unspezifische Sensoren, die bei Anwesenheit von gasförmigen Verbindungen ihren elektrischen Widerstand ändern. Die Sensoren sind jedoch nicht identisch. Sie unterscheiden sich durch die Verwendung unterschiedlicher Halbleiter, durch unterschiedliche Dotierungen und durch verschiedene Betriebstemperaturen. Die Sensoren liefern daher unterschiedliche Signale bei entsprechenden gasförmigen Verbindungen. Die Nachweisgrenzen der Sensoren liegen im ppb- bzw. ppm-Bereich, d.h. im Bereich von 1:10⁹ bzw. 1:10⁶, oder auch darunter, bspw. im Bereich von 1:10¹⁰ bis 1:10¹².

Die Intensität der Sensorsignale verhält sich proportional zur Konzentration der Geruchsstoffe, insbesondere von organischen Verbindungen, in der Luft. Somit kann man nicht nur qualitative sondern auch quantitative Aussagen über die vom Körper abgesonderten Geruchsstoffe treffen.

Die Sensoren eines Sensorarrays liefern bei Vorliegen einer gasförmigen Verbindung ein charakteristisches Muster. Bei einem Vergleich mit vorherigen Mustern können somit bestimmte Stoffe identifiziert werden. Es lassen sich aber auch bei einem Vergleich mit vorher ermittelten Messwerten Aussagen über die Veränderung der Zusammensetzung eines Geruchs treffen. Insbesondere kann man feststellen, ob einzelne Verbindungen in ihrer Konzentration zunehmen oder abnehmen.

Das Messgas gelangt zur Untersuchung für einen kurzen Zeitraum von bspw. 20 sec. in eine Sensorkammer, die eine Größe von etwa 1 bis 2 ml aufweisen kann. Nach erfolgter Messung kann die Messkammer mit gereinigter Luft gegengespült werden und zwar vorzugsweise für eine Dauer von ca. 40 sec. Die Spülluft kann aus der Umgebungsluft gewonnen werden, indem sie mit einem Aktivkohlefilter gereinigt wird. Vorzugsweise ist auch dieser Filter im Gehäuse der Sensoreinheit untergebracht.

Aufgrund der Empfindlichkeit der Sensoren kommt man gegebenenfalls jedoch auch ohne Messkammer und Reinigung der Messkammer aus, indem der Sensor einfach in der Umgebungsluft des Probanden angebracht wird. In diesem Fall sind die Messergebnisse jedoch weniger genau, dafür ist jedoch die Vorrichtung insgesamt weniger aufwendig, kleiner und weniger störanfällig.

Mit einer Aufzeichnungseinheit werden die fortlaufend gemessenen Signale protokolliert. Die Protokollierung kann entweder auf einem elektronischen Datenträger oder in einfacheren Fällen auf einem elektro-mechanischen Schreiber erfolgen. Durch diese fortlaufende Aufzeichnung lässt sich eine Tendenz, insbesondere die Zunahme oder Abnahme einer Substanz feststellen.

Die beschriebene elektronische Sensoreinheit ist in der Lage, auch komplexe Gemische aus gasförmigen Verbindungen zu erkennen. Insbesondere können Abweichungen von Normwerte sofort erfasst werden. Die Messwerte können über mathematische Methoden weiterverarbeitet werden. Hierbei werden insbesondere Distanzklassifikatoren (Euklid, Korrelation), Faktorenanalyse oder künstliche neuronale Netze (Koronen) verwendet.

Die elektronische Sensoreinheit hat gegenüber der menschlichen Nase, den Vorteil, dass sie wesentlich empfindlicher ist und die Ergebnisse objektiv sind, d.h. nicht von der Tagesform eines Menschen abhängen. Ferner weist die elektronische Sensoreinheit auch keine Ermüdungserscheinungen auf.

Die beschriebene Sensoreinheit wird in einem solchen Bereich der zu untersuchenden Person vorgesehen, dass sie die Person nicht beeinflusst bzw. stört. Die Sensoreinheit ist dazu von der Person überhaupt nicht wahrnehmbar. Vorzugsweise ist die Sensoreinheit im Bereich eines Bettes angeordnet. Hierbei eignet sich vor allem eine Position an oder in der Matratze bzw. dem Bettgestell oder zwischen der Matratze und einem Oberbett. Vorteilhafterweise werden mehrere Sensoreinheiten an unterschiedlichen Positionen angeordnet, und zwar insbesondere über die Fläche des Betts oder die Matratze gleichmäßig oder auch ungleichmäßig verteilt. Diese Verteilung erfolgt in der Regel so, dass der Gesamtkörpergeruch ermittelbar ist, wobei solchen Stellen des Körpers, die besonders charakterische Gerüche erwarten lassen, gezielt Gassensoren zugeordnet sind. Dadurch kann man verschiedene Geruchsquellen am ganzen Körper der Person lokalisieren, es sind Aussagen über den Ort einer möglicherweise entstehenden oder bereits akuten Entzündung oder dergleichen am Probanden möglich.

Die erhaltenen Messwerte werden entweder direkt oder nach einer Vorverarbeitung, bspw. unter Verwendung o.g. mathematischer Methoden, mittels einer Anzeigeeinheit zur Anzeige gebracht. Diese Anzeige erlaubt einer mit dem Monitoringsystem vertrauten Person die gemessenen Werte als Zwischenergebnis für eine zu stellende Diagnose zu verwerten. Außerdem kann, falls ein oder mehrere Sensorsignale einen Grenzwert erreichen bzw. über- oder unterschreiten, ein Alarmsignal ausgelöst werden, um eine Pflegeperson herbeizurufen.

Es ist auch möglich, die geruchsbezogenen Messwerte zusammen mit anderen Messwerten oder Signalen auszuwerten. Beispielsweise können Drücke wie Auflagedrücke der Person auf der Matratze oder einer anderen Komponente eines Bettes, Liege oder dergleichen mit den Geruchsmesswerten verglichen werden. Solche Drücke bzw. Auflagerkräfte werden vorzugsweise dort ermittelt, wo auch der Geruch festgestellt wird, also in Bereichen aller oder ausgewählter Geruchssensoren.

Mittels der Erfindung ist es möglich, physiologische bzw. chemische oder physikalische Zustände an einem unversehrten Körper nicht-invasiv zu ermitteln. Ein Eingriff an dem Probanden ist daher nicht notwendig. Die Untersuchung erfolgt vielmehr berührungslos. Dieser Vorteil erlaubt es, die Messungen fortlaufend und langandauernd, praktisch über Tage bzw. Wochen oder Monate durchzuführen, ohne den Probanden dabei zu beeinträchtigen.

Mit dem beschriebenen olfaktorischen Monitoringsystem lässt sich insbesondere im Pflegebereich die Qualität der Pflege beobachten, und zwar ohne Einbeziehung, das heißt mit Hilfe und/oder Beeinträchtigung der betreffenden Person. Versäumt es beispielsweise ein Pfleger eine bettlägerige Person regelmäßig zu bewegen, kommt es zum Dekubitus. Mit dem beschriebenen Monitoringsystem, kann ein sich anbahnender Dekubitus jedoch bereits im Vorfeld erkannt und somit seine Ausbildung verhindert werden. Insbesondere kann man mit dem beschriebenen System jedoch feststellen, ob das Pflegepersonal seinen Pflichten nachkommt. Dies erlaubt eine objektive Bewertung der Qualität einer Pflegeeinrichtung. Die Erfindung hat somit auch vielfältige Einsatzmöglichkeiten im nicht-therapeutischen Bereich.

## Patentansprüche

1. Vorrichtung zur Untersuchung und/oder Protokollierung von menschlichen Erkrankungen, Körperzuständen und/oder physiologischen Prozessen einer Person, mit einer die Person unbeeinflussenden Geruchssensoreinheit und einer Anzeigeeinheit zur Anzeige eines von der Geruchssensoreinheit erzeugten Sensorsignals, wobei die Geruchssensoreinheit im Bereich der Person anbringbar ist, **dadurch gekennzeichnet, dass** mehrere Geruchsensoreinheiten im Bereich einer Matratze oder einem Bettgestell in der Regel derart flächig verteilt sind, dass der Gesamtkörpergeruch der Person ermittelbar ist, und dass solchen Stellen des Körpers, die charakteristische Gerüche erwarten lassen, gezielt Geruchssensoreinheiten zugeordnet sind.

2. Vorrichtung nach Anspruch 1, **gekennzeichnet durch** eine Aufzeichnungseinheit zum insbesondere fortlaufenden Aufzeichnen von Sensorsignalen.

3. Vorrichtung nach Anspruch 2, **gekennzeichnet durch** eine Auswerteeinheit zum Auswerten eines aktuellen oder aufgezeichneten Sensorsignals, insbesondere Vergleichen eines Werts des Sensorsignals mit weiteren zuvor aufgezeichneten Werten und zur Ausgabe eines Resultats, insbesondere eines eine Verschlechterung, eine Besserung oder einen gleichbleibenden Zustand anzeigenden Resultats.

4. Vorrichtung nach einem der Ansprüche 1 bis 3, **dadurch gekennzeichnet, dass** die Geruchssensoreinheit mehrere Gassensoren, zum Beispiel Halbleitergassensoren, aufweist.

5. Vorrichtung nach einem der vorhergehenden Ansprüche, **dadurch gekennzeichnet, dass** einige oder alle Gassensoren der Geruchssensoreinheit unterschiedlich auf Geruchsstoffe reagieren, insbesondere ihren elektrischen Widerstand ändern.

6. Vorrichtung nach einem der vorhergehenden Ansprüche, **dadurch gekennzeichnet, dass** die Gassensoren unterschiedliche Halbleiter, Dotierungen und/oder Betriebstemperaturen aufweisen.

7. Vorrichtung nach einem der vorhergehenden Ansprüche, **dadurch gekennzeichnet, dass** die Auswerteeinheit eine Vergleichseinheit aufweist, um Muster der Sensorsignale mit vorher gespeicherten Mustern, insbesondere aufgezeichneten Mustern, zu vergleichen.

8. Verfahren zur Untersuchung und/oder Protokollierung von menschlichen Erkrankungen, Körperzuständen und/oder physiologischen Prozessen einer Person, wobei eine Geruchsprobe mittels einer Geruchssensoreinheit untersucht und ein von der Geruchssensoreinheit erzeugtes Sensorsignal angezeigt wird, wobei die Geruchssensoreinheit im Bereich der Person angebracht wird, **dadurch gekennzeichnet, dass** mit mehreren Geruchssensoreinheiten der Geruch des im Wesentlichen ganzen Körpers der Person im Bereich einer Matratze oder einem Bettgestell ermittelt wird, und solchen Stellen des Körpers, die besonders charakteristische Gerüche erwarten lassen, gezielt Geruchssensoreinheiten zugeordnet werden.

9. Verfahren nach Anspruch 8, **dadurch gekennzeichnet, dass** das Sensorsignal fortlaufend aufgezeichnet wird, insbesondere das Sensorsignal mit einem zuvor aufgezeichneten Sensorsignal oder einem Referenzsignal verglichen wird, um eine Tendenz des Sensorsignals festzustellen.

10. Verfahren nach einem der Ansprüche 8 oder 9, **dadurch gekennzeichnet, dass** einige oder alle Geruchsproben mit anderen Messwerten, zum Beispiel auf den Körper einwirkenden Drücken, verglichen werden.

11. Verfahren nach einem der vorhergehenden Ansprüche, **dadurch gekennzeichnet, dass** das Sensorsignal bei Erreichen bzw. Über- oder Unterschreiten eines Grenzwerts ein Alarmsignal auslöst.

12. Verwendung einer Vorrichtung nach einem oder mehreren der Ansprüche 1 bis 7, zur Untersuchung und/oder Protokollierung von menschlichen Krankheiten, insbesondere zur Prophylaxe und/oder Früherkennung von menschlichen Krankheiten.

13. Verwendung einer Vorrichtung nach einem oder mehreren der Ansprüche 1 bis 7, zur Untersuchung und/oder Protokollierung von Entzündungen im kutanen und/oder subkutanen Bereich des insbesondere menschlichen Körpers.

14. Verwendung einer Vorrichtung nach einem oder mehreren der Ansprüche 1 bis 7, zur Ermittlung des Immunstatus und/oder der Aktivität des Immunsystems und/oder virale Erkrankungen und/oder bakterielle Erkrankungen des menschlichen Körpers.

## Claims

1. Device for examining and/or recording medical disorders, the physical condition and/or physiological processes of a person, with an odor sensor unit device, which does not affect the person, and a display unit for indicating a sensor signal generated by the odor sensor unit, it being possible to place the odor sensor unit in the region of the person, **characterized in that** a plurality of odor sensor units positioned in the region of a mattress or bed frame are distributed over a significant surface area in order to record the overall body odor of the person, and that odor sensor units are specifically assigned to those parts of the body where characteristic odors can be expected.

2. Device according to Claim 1, **characterized by** a recording unit for the in particular continuous recording of sensor signals.

3. Device according to Claim 2, **characterized by** an evaluation unit for evaluating a current or recorded sensor signal, in particular for comparing the value of a sensor signal with other previously recorded values, and for the output of a result, in particular a result that indicates a worsened, improved or stabilized condition.

4. Device according to one of the Claims 1 to 3, **characterized in that** the odor sensor unit has a plurality of gas sensors, for example semiconductor gas sensors.

5. Device according to one of the previous Claims, **characterized in that** some or all gas sensors of the odor sensor unit react differently to odorants, in particular by altering their electric resistance.

6. Device according to one of the preceding Claims, **characterized in that** the gas sensors exhibit different semiconductors, dopant concentrations and/or operating temperatures.

7. Device according to one of the preceding Claims, **characterized in that** the evaluation unit has a comparison unit for comparing samples of the sensor signals of a sensor array with previously stored samples, in particular recorded samples.

8. Method for examining and/or recording medical disorders, the physical condition and/or physiological processes of a person, with an odor sample being examined by means of an odor sensor unit and a sensor signal generated by the odor sensor unit being displayed, with the odor sensor unit being positioned in the region of the person, **characterized in that** the odor of essentially the entire body of the person is determined by a plurality of sensor units positioned in the region of a mattress or bed frame, and that odor sensor units are specifically assigned to those parts of the body where characteristic odors can be expected.

9. Method according to Claim 8, **characterized in that** the sensor signal is recorded continuously, in particular the sensor signal is compared with a previously recorded sensor signal or reference signal, in order to establish a tendency in the sensor signal.

10. Method according to Claims 8 or 9, **characterized in that** that some of all odor samples are compared to other measured values, for example pressure forces exerted on the body.

11. Method according to one of the preceding Claims, **characterized in that** the sensor signal triggers an alarm signal upon reaching, exceeding or not reaching a limit value.

12. Application of a device according to one or more of the Claims 1 to 7, for the purpose of examining and/or recording medical disorders in humans, in particular for the prophylactics and/or early detection of human illnesses.

13. Application of a device according to one or more of the Claims 1 to 7, for the purpose of examining and/or recording inflammations in the cutaneous and/or subcutaneous region of the in particular human body.

14. Application of a device according to one or more of the Claims 1 to 7, for the purpose of determining the immune status and/or activity of the immune system and/or viral disorders and/or bacterial disorders of the human body.

## Revendications

1. Dispositif pour analyser et/ou consigner des maladies humaines, états corporels et/ou processus physiologiques d'une personne, comprenant une unité de détection d'odeur qui n'influence pas la personne et une unité d'affichage pour afficher un signal de capteur généré par l'unité de détection d'odeur, l'unité de détection d'odeur pouvant être amenée à proximité de la personne, **caractérisé en ce que** plusieurs unités de détection d'odeur sont réparties dans la zone d'un matelas ou d'un châssis de lit généralement à plat de manière à ce qu'il soit possible de déterminer l'odeur corporelle totale de la personne et que des unités de détection d'odeur soient affectées aux endroits du corps où l'on peut s'attendre à des odeurs caractéristiques.

2. Dispositif selon la revendication 1, **caractérisé par** une unité d'enregistrement notamment destinée à l'enregistrement continu de signaux de capteurs.

3. Dispositif selon la revendication 2, **caractérisé par** une unité d'interprétation destinée à interpréter un signal de capteur actuel ou enregistré, notamment à comparer une valeur du signal de capteur avec d'autres valeurs.enregistrées précédemment et à délivrer un résultat, notamment un résultat indiquant une dégradation, une amélioration ou un état stationnaire.

4. Dispositif selon l'une des revendications 1 à 3, **caractérisé en ce que** l'unité de détection d'odeur présente plusieurs capteurs de gaz, par exemple des capteurs de gaz à semi-conducteur.

5. Dispositif selon l'une des revendications précédentes, **caractérisé en ce que** certains ou la totalité des capteurs de gaz de l'unité de détection d'odeur réagissent différemment aux substances odorantes, notamment en modifiant leur résistance électrique.

6. Dispositif selon l'une des revendications précédentes, **caractérisé en ce que** les capteurs de gaz présentent des semi-conducteurs, des dopages et/ou des températures de fonctionnement différents.

7. Dispositif selon l'une des revendications précédentes, **caractérisé en ce que** l'unité d'interprétation présente une unité de comparaison afin de comparer des modèles des signaux de capteurs avec des modèles mémorisés précédemment, notamment des modèles enregistrés.

8. Procédé pour analyser et/ou consigner des maladies humaines, états corporels et/ou processus physiologiques d'une personne, un échantillon d'odeur étant analysé au moyen d'une unité de détection d'odeur et un signal de capteur généré par l'unité de détection d'odeur étant affiché, l'unité de détection d'odeur étant amenée à proximité de la personne, **caractérisé en ce que** l'odeur du corps de la personne essentiellement dans son ensemble est déterminée par plusieurs unités de détection d'odeur dans la zone d'un matelas ou d'un châssis de lit et **en ce que** des unités de détection d'odeur sont affectées aux endroits du corps où l'on peut s'attendre à des odeurs particulièrement caractéristiques.

9. Procédé selon la revendication 8, **caractérisé en ce que** le signal de capteur est enregistré continuellement, le signal de capteur est notamment comparé avec un signal de capteur enregistré précédemment ou avec un signal de référence afin de déterminer une tendance du signal de capteur.

10. Procédé selon l'une des revendications 8 ou 9, **caractérisé en ce que** certains ou la totalité des échantillons d'odeur sont comparés avec d'autres valeurs mesurées, par exemple des pressions agissant sur le corps.

11. Procédé selon l'une des revendications précédentes, **caractérisé en ce que** le signal de capteur déclenche une alarme en atteignant ou encore en franchissant dans un sens ou dans l'autre une valeur de seuil.

12. Utilisation d'un dispositif selon une ou plusieurs des revendications à 1 à 7 pour analyser et/ou consigner des maladies humaines, notamment pour la prophylaxie et/ou le diagnostic anticipé de maladies humaines.

13. Utilisation d'un dispositif selon une ou plusieurs des revendications à 1 à 7 pour analyser et/ou consigner des inflammations dans la zone cutanée et/ou sub-cutanée notamment du corps humain.

14. Utilisation d'un dispositif selon une ou plusieurs des revendications à 1 à 7 pour déterminer l'état immunitaire et/ou l'activité du système immunitaire et/ou des maladies virales et/ou des maladies bactériennes du corps humain.
